(19) Europäisches Patentamt
European Patent Office
Office européen des brevets

(11) Publication number : **0 245 093 B1**

(12) # EUROPEAN PATENT SPECIFICATION

(45) Date of publication of patent specification : **11.03.92 Bulletin 92/11**

(51) Int. Cl.$^5$ : **C12Q 1/00,** C12N 15/12

(21) Application number : **87304051.3**

(22) Date of filing : **06.05.87**

(54) **Method for detecting metals.**

(30) Priority : **08.05.86 JP 103849/86**

(43) Date of publication of application : **11.11.87 Bulletin 87/46**

(45) Publication of the grant of the patent : **11.03.92 Bulletin 92/11**

(84) Designated Contracting States : **DE FR GB**

(56) References cited :
**EP-A- 0 137 515**
**EP-A- 0 187 519**
**TRENDS IN ANALYTICAL CHEMISTRY, vol. 1, no. 16, December 1982, pages 378-383, Elsevier Scientific Publishing Co., Cambridge, GB; F.G. PRENDERGAST: "The use of photoproteins in the detection and quantitation of Ca2+ in biological systems"**

(56) References cited :
**BIOLOGICAL ABSTRACTS, vol. 80, no. 8, 1985, page 474, abstract no. 68408, Philadelphia, Pasadena, US; O. SHIMOMURA et al.: "Halistaurin, phialidin and modified forms of aequorin as calcium indicator in biological systems", & BIOCHEM J. 228(3), 745-750, 1985**
**CHEMICAL ABSTRACTS, vol. 79, no. 13, 1st October 1973, page 185, abstract no. 75180y, Columbus, Ohio, US; O. SHIMOMURA et al.: "Further data on the specificity of aequorin luminescence to calcium", & BIOCHEM. BIOPHYS. RES. COMMUN. 1973, 53(2), 490-4**
**BIOCHEMISTRY, vol. 25, no. 26, 30th December 1986, pages 8425-8429, American Chemical Society, Easton, PA, US; S. INOUYE et al.: "Expression of apoaequorin complementary DNA in Escherichia coli"**

(73) Proprietor : **CHISSO CORPORATION**
**6-32, Nakanoshima 3-chome Kita-ku**
**Osaka-shi Osaka 530 (JP)**

(72) Inventor : **Inouye, Satoshi 103 Haitsu Sumi, 1-7**
**Teramae 1-chome Kanazawa-ku**
**Yokohama-shi Kanagawa-ken (JP)**

(74) Representative : **Ackroyd, Robert et al**
**W.P. THOMPSON & CO. Eastcheap House**
**Central Approach**
**Letchworth, Hertfordshire SG6 3DS (GB)**

**EP 0 245 093 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a method for detecting metals and especially to such a method in which a photoprotein is used, namely an enzyme of aequorin (apoaequorin), together with a substrate, as an emitter, namely coelenterazine or its analogues.

Aequorin is a photoprotein obtainable from luminescent jellyfish which grow in the northern part of the west coast of America. When one molecule of aequorin is specifically bound to two or three molecules of $Ca^{2+}$, it oxidises the coelenterazine present, which acts as an emitter to produce luminescence. Thus, natural aequorin exists in the package state containing the enzyme (apoaequorin), the substrate (coelenterazine) and molecular oxygen ($O_2$). When $Ca^{2+}$ is added to natural aequorin, its reaction with the $Ca^{2+}$ -binding sites present causes the coelenterazine to be oxidised, so that oxidizedcoelenterazine, $CO_2$ and hν (emission) are produced. This light emission can be detected by a photomultiplier and its sensitivity is so high that trace concentrations of $Ca^{2+}$ of about $10^{-7}$M are measurable.

The habitat of the jellyfish which produce natural aequorin is limited to the aforesaid coastal region and the season in which they are produced is also limited. The yield of natural aequorin is only about 10 mg per 50,000 jellyfish. It is thus very important to obtain aequorin, but a certain supply cannot be assured.

The use of photoproteins such as natural aequorin in the detection and quantitation of $Ca^{2+}$ ions in biological systems is described by F G Prendergast in Trends in Analytical Chemistry 1, 378-382 (1982).

EP-A-0137515 describes the production of blue light by addition of $Ca^{2+}$ to solutions of coelenterate-derived proteins, and the recharging of aphotoproteins in the absence of $Ca^{2+}$.

The inventor of the present invention has cloned the cDNA for aequorin obtained from these jellyfish by a recombinant DNA method. This cDNA clone was named plasmid pAQ440 (JP-A-59-176125). Then, a plasmid containing a promoter in which the pAQ440 gene was inserted was transformed in a bacterium and it was found that the natural and fused type of aequorin (apoaequorin) could be efficiently expressed in E.coli (JP-A-60-280259). This method provided a stable supply of aequorin.

The present invention further improves the method for detecting $Ca^{2+}$ concentrations using natural aequorin and also provides a method for detecting metals, such as $Ca^{2+}$ and others, using apoaequorin biosynthesized in E.coli.

According to one aspect of the present invention, there is provided a method for detecting a metal in a sample, comprising adding aequorin to the sample and measuring the luminescence of the aequorin produced in order to detect the metal, characterized in that the aequorin has been regenerated by adding coelenterazine or an analogue thereof to an apoaequorin produced in E.coli by a biosynthetic method.

Preferably, in carrying out the method of the invention, the apoaequorin is produced using the apoaequorin cDNA obtained by the biosynthetic method disclosed in the above-mentioned patent application and then, by adding coelenterazine as a substrate to the apoaequorin, aequorin having the same properties as natural aequorin is obtained. The luminescence shown by various metals when reacted with this synthesized aequorin is measured, in order to detect and/or measure the amount of such metals. As a result, the presence of $Ca^{2+}$, $Sr^{2+}$ and $Sm^{3+}$ can be observed. For $Cd^{2+}$, its presence can be confirmed from the inhibitory rate of luminescence of $Ca^{2+}$. Lanthanoids such as $La^{3+}$, $Tb^{3+}$ and $Yb^{3+}$ also can be detected.

The following description serves to illustrate the invention more specifically.

(1) A method for producing aequorin (apoaequorin) in E.Coli.

(1) Insertion of aequorin genes into an expression vector having a promoter.

As the expression vector into which the aequorin gene is inserted, the vector in which the promoter is cloned is used. As the expression vector, pUC9 can be cited as an example. As the promoter, lac, tac or trp derived from E.coli or PL of the λ phage can be used, by way of example.

Firstly, pDR540 having the promoter is digested by means of the restriction enzyme BamHI-HindIII. The resulting fragment containing the promoter is then separated and extracted by an electrophoresis method, e.g. in 8% acrylamide gel. By using the promoter thus extracted, the expression vector (piC9) can be obtained by cloning at the BamHI-HindIII site of the vector.

Then, after digesting this expression vector with HindIII, its terminals are repaired by E.coli DNA polymerase (Klenow fragment) in the presence of dATP, dGTP, dCTP and dTTP, both the terminals are ligated with $T_4$ ligase and an expression vector, piC10, is thus obtained.

In preparing to bind the promoter in the piC10 with the aequorin cDNA gene, a synthetic nucleotide as a linker is cloned to piC10. Thus, the synthetic nucleotide linkers AR(5′GATCGATGGTCA-3′) and AQ(5′AGCTTGACCATC-3′) are first prepared, by a known synthetic method, and then annealed, after which

phosphorylation of their 5'-terminals is carried out using $T_4$ nucleotide kinase in the presence of ATP. The nucleotides thus phosphorylated are subsequently cloned, so as to construct plasmids in the form of repetition units of the linker at the BamHI-EcoRI site of the piC10, which has been constructed as mentioned above, and a recombinant DNA piC11 is so obtained.

Next, a HindIII-EcoRI fragment is separated from aequorin cDNA clone pAQ440 (see Japanese Patent Application 59-176125), this fragment is then inserted into the HindIII-EcoRI site of the above piC11, and the desired piQ5, namely bacteria containing a promoter in which the apoaequorin gene has been transformed, is obtained. This piQ5 has the ability to produce aequorin proteins of natural type.

(2) Insertion of aequorin genes into an expression vector (pUC9, 9-1, 9-2) having a lac promoter in order to produce aequorin proteins of a fused type.

In a similar manner to that described in (1) above, PstI-EcoRI and HindIII-EcoRI fragments are separated from the cDNA clone pAQ440 and purified. Further, expression vectors pUC9, 9-1, 9-2 having a lac promoter prepared by a similar method to that described in (1) above are obtained. Each of these fragments is cloned at the restriction enzyme site of each of these vectors to produce piQ9PE, 9-1PE, 9-2PE,9-HE and 9-2HE. All of these piQ plasmids are under the control of the lac promoter and thus comprise aequorin proteins of the fused type having 8-amino acid residues at an N-terminus.

(3) Production of proteins having aequorin activity using E.coli.

Each plasmid cloned to the expression vectors obtained by the methods of (1) and (2) is transformed in a strain, such as E.coli (D 1210), and aequorin proteins are produced. In this method, for example, E.coli strains containing plasmids are added to a certain volume of LB broth containing a given concentration of ampicillin and the E.coli strains are cultured. Subsequently, an expression-inducible reagent is added to the culture medium and incubation is continued. The resulting culture medium is separated by centrifugation and the resulting cells are collected and washed with water.

(2) A method for the synthesis of coelenterazine and its analogues.

In this method, coelenterazine obtained by a known method can be used.

The coelenterazine used here has the following general formula:

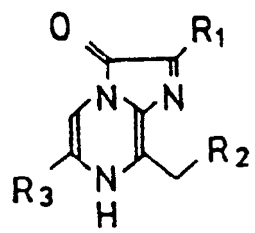

(I)

Coelenterazine of the natural type is represented by the formula wherein $R_1$ is p-$CH_2C_6H_4OH$, $R_2$ is -$C_6H_5$ and $R_3$ is p-$C_6H_4OH$.

Coelenterazine compounds represented by the formula (I) can be obtained by a known method, for example, the method disclosed by Inoue et al. (Published by Japan Chemical Society, Chemistry Letters, 141-144, 1975).

Further, as analogues of coelenterazine, analogue 1, represented by the formula (I) wherein $R_1$ is -$CH_2C_6H_5$, $R_2$ is -$C_6H_5$ and $R_3$ is p-$C_6H_4OH$, or analogue 2, represented by the formula (I) wherein $R_1$ is -$CH_3$, $R_2$ is -$C_6H_5$ and $R_3$ is p-$C_6H_4OH$, can be used. These analogues can be obtained by a known method, for example, the method disclosed by Halt et al. (Biochemistry, 18, (11) 2204-2210, 1979).

(3) A method for detecting metals.

Metals are detected by the following method, for example.

The enzyme solution obtained by extraction from E.coli using the above process (3) is used. After dissolving the enzyme solution in Tris-HCl buffer solution containing EDTA, coelenterazine as a substrate and 2-mercaptoethanol are added to the solution and it is then allowed to stand on ice, while aequorin is reproduced. A sample

to be analysed is poured into the solution containing aequorin thus reproduced, and the resulting mixed solution is transferred to the reaction cell of a spectro-photometer. A sample to be measured is then injected into the cell and the quantity of luminescence produced is measured.

By using this invention, metals can be detected by means of aequorin produced in E.coli by the recombinant DNA method, using coelenterazine or its analogues as the substrate. Therefore, without using natural aequorin, which is difficult to obtain, metals can be detected by readily available synthetic aequorin produced in E.coli, and so can be easily, efficiently and economically detected.

The following non-limitative examples illustrate this invention more specifically.

EXAMPLE 1

Production of aequorin (apoaequorin) from E.coli

Construction of piC10:

The plasmid pDR540 having a tac promoter (produced by PL Pharmacia Co. Ltd.) was digested by the restriction enzyme BamHI-HindIII and then the resulting fragment containing the tac promoter of 92bp was separated and extracted by the electrophoresis method using acrylamide. Then, the BamHI-HindIII site of the resultant vector pUC9 was cloned and piC9 was constructed. Then, in oder to remove the HindIII site of the expression vector piC9, firstly the vector MiC9 was digested with HindIII and then, after repairing the terminals of the vector by E.coli DNA polymerase (Klenow fragment) in the presence of dATP, dGTP, dCTP and dTTP, both the terminals were ligated with $T_4$ ligase and piC10 was thus constructed.

EXAMPLE 2

Construction of piC11:

In order to bind the aequorin cDNA gene and the promoter, synthetic linker nucleotides of AR(5′GATCGATGGTCA-3′) and AQ(5′AGCTTGACCATC-3′) were prepared. After these nucleotides were annealed, the 5′ terminals were phosphorylated with $T_4$ nucleotide kinase in the presence of ATP. The nucleotides thus phosphorylated were subsequently cloned, so as to construct in the form of repetition units of the linker at the BamHI-EcoRI site of the piC10 (EXAMPLE 1), and piC11 was constructed.

EXAMPLE 3

Construction of piQ5:

The HindIII-EcoRI fragment was separated and prepared from aequorin cDNA clone pAQ440, the fragment was inserted into the HindIII-EcoRI site of the above piC11, and piQ5 was thus obtained. This piQ5 can reproduce aequorin protein of the natural type.

EXAMPLE 4

Construction of piQ9PE, 9-1PE, 9-2PE, 9-HE and 9-2HE

After PstI-EcoRI and HindIII-EcoRI fragments were separated and purified from cDNA clone pAQ440, these fragments were cloned at the restriction enzyme site of expression vectors pUC9, 9-1, 9-2, having a lac promoter, and piQ9PE, 9-1PE, 9-2PE, 9-HE and 9-2HE were constructed. All of these piQ fragments were under the control of the lac promoter and so comprised aequorin proteins of the fused type having 8-amino-acid residues at an N-terminus.

EXAMPLE 5

Production of proteins having aequorin activity using E.coli:

The above E.coli strains containing plasmids and having 1/100 volumes, obtained by culturing for 12 hours, were added to 10 ml of LB broth containing 50 μg/ml of ampicillin. The E.coli strains were cultured for 2 hours at 37°C, subsequently an expression-inductible reagent IPTG (isopropyl-β-thiogaractopyranoside) was added, to obtain a final concentration of 1 mM, and incubation was continued for 4 hours at 42°C.

The resultant culture medium was separated by centrifugation at 5060 rpm for 10 minutes (Hitachi RP 20) and the cells were collected and washed with 5 ml of M9 salt medium. After resolving the washed cells in 2.5 ml of 20 mM Tris-HCl buffer (pH 7.6) containing 10 mM of EDTA, the cells were destroyed by sonication (60 seconds), the mixture was centrifuged at 10,000 rpm for 10 minutes and the resulting supernatent was used as an enzyme solution for detecting metals.

EXAMPLE 6

Synthesis of coelenterazine and its analogues

Coelenterazine and its analogues were synthetized by the method discloses by Inoue et al. (Published by Japan Chemical Society, Chemistry Letters, 141-144, 1975) and by the method disclosed by Halt et al. (Biochemistry, 18, (11) 2204-2210, 1979).

EXAMPLE 7

Method for detecting metals

The enzyme solution obtained by the above method was dissolved in 30 mM of Tris-HCl buffer soution containing 10 mM of EDTA, 1 μg of the same type of coelenterazine substrate as the natural type, obtained by the synthesis method disclosed by Inoue et al., and 5 μl of 2-mercaptoethanol were added to the solution to obtain 1 ml of total volume. The solution was allowed to stand for 1 hour in an ice bath and aequorin was reproduced. Then, the concentration of metals was measured.

Each sample containing $CaCl_2$, $SrCl_2$ and $SmCl_3$ (1.5 ml, 30 mM) was poured into the solution of aequorin thus reproduced, the solution was transferred to the reaction cell of a spectrophotometer (Mitchell-Hasting photometer) and the luminescence of the sample was measured (1). Further, 30 mM of $CaCl_2$ was injected into the cell and the luminescence was measured again (2).

The results are shown in the following table:

| Metal ion | (1) | (2) |
|---|---|---|
| $Ca^{2+}$ | 8.3 | 0 |
| $Sr^{2+}$ | 4.9 | 0 |
| $Sm^{3+}$ | 3.6 | 0 |
| $Mn^{2+}$ | 0 | 8.8 |
| $Mg^{2+}$ | 0 | 8.4 |
| $Pb^{2+}$ | 0 | 8.3 |
| $Cd^{2+}$ | 0 | 0.9 |

In the table, the units of the numerals are $x10^{-11}$ photo/sec.

It is apparent from the above table that the metal ions $Ca^{2+}$, $Sr^{2+}$ and $Sm^{3+}$ can be detected and the presence of $Cd^{2+}$ can be also detected because the inhibitory rate of $Cd^{2+}$ is 90%.

Metals were detected using synthesised coelenterazine of the natural type in the above examples. Further, when the metals were detected by using analogues of coelenterazine, similar results were obtained.

## Claims

1. A method for detecting a metal in a sample, comprising adding aequorin to the sample and measuring the luminescence of the aequorin produced in oder to detect the metal, characterized in that the aequorin has been regenerated by adding coelenterazine or an analogue thereof to an apoaequorin produced in E.coli by a biosynthetic method.

2. A method as claimed in claim 1, wherein the biosynthetic method is a recombinant DNA technique and the apoaequorin is produced by using apoaequorin cDNA.

3. A method as claimed in claim 1 or 2, wherein the metal is $Ca^{2+}$, $Sr^{2+}$, $Sm^{3+}$, $Cd^{2+}$ or a lanthanoid.

4. A method as claimed in claim 3, wherein the lanthanoid is $La^{3+}$, $Tb^{3+}$ or $Yb^{3+}$.

## Patentansprüche

1. Verfahren zum Nachweis eines Metalles in einer Probe, umfassend die Zugabe von Aequorin in die Probe und Messen der Luminiszenz des erzeugten Aequorins zum Nachweis des Metalles, **dadurch gekennzeichnet** , daß das Aequorin durch Zusatz von Coelenterazin oder eines Analoges davon in ein durch ein biosynthetisches Verfahren in E. coli erzeugtes Apoaequorin regeneriert wurde.

2. Verfahren nach Anspruch 1, worin das biosynthetische Verfahren eine rekombinante DNS-Technik ist und das Apoaequorin unter Verwendung von Apoaequorin-cDNS hergestellt wird.

3. Verfahren nach Anspruch 1 oder 2, worin das Metall $Ca^{2+}$, $Sr^{2+}$, $Sm^{3+}$, $Cd^{2+}$ oder ein Seltenerdmetall ist.

4. Verfahren nach Anspruch 3, worin das Seltenerdmetall $La^{3+}$, $Tb^{3+}$ oder $Yb^{3+}$ ist.

## Revendications

1. Procédé de détection d'un métal dans un échantillon, qui consiste à ajouter de l'aequorine à l'échantillon et à mesurer la luminescence de l'aequorine produite, pour détecter le métal, caractérisé en ce que l'aequorine a été régénérée par addition de coelentérazine ou d'un de ses analogues à une apoaequorine produite par *E.coli* par un procédé biosynthétique.

2. Procédé selon la revendication 1, dans lequel le procédé biosynthétique est une technique de recombinaison d'ADN et l'apoaequorine est produite en utilisant de l'ADNc d'apoaequorine.

3. Procédé selon la revendicatlon 1 ou 2, dans lequel le métal est $Ca^{2+}$, $Sr^{2+}$, $Sm^{3+}$, $Cd^{2+}$ ou un lanthanide.

4. Procédé selon la revendication 3, dans lequel le lanthanide est $La^{3+}$, $Tb^{3+}$ ou $Yb^{3+}$.